# EUROPEAN PATENT SPECIFICATION

(11) **EP 0 308 188 B1**
(45) Date of publication and mention of the grant of the patent: **08.05.1996**
(21) Application number: 88308468.3
(22) Date of filing: 14.09.1988
(51) Int. Cl.: C07D 301/32, C07D 301/19, C07D 303/14

(54) **Chiral epoxyalcohol recovery in asymmetric epoxidation process**
Abtrennung von chiralen Epoxyalkoholen in asymmetrischen Epoxidierungsverfahren
Séparation d'époxyalcools chiraux dans un procédé d'époxydation asymétrique

(30) Priority: 14.09.1987 US 95736
(43) Date of publication of application: 22.03.1989
(73) Proprietor: ARCO Chemical Technology, L.P., Greenville, Delaware 19807 (US)
(72) Inventor: Shum, Wilfred Po-sum, Swarthmore Pennsylvania 19081 (US)
(74) Representative: Cropp, John Anthony David

(56) References cited:
- EP-A- 0 046 033
- FR-A- 1 462 491
- US-A- 3 374 153

## Description

### BACKGROUND OF THE INVENTION

The present invention is directed to an improvement in the process of asymmetric epoxidation of allylic alcohols. More specifically, the invention is directed to an improved method of recovery of the epoxidized product.

Epoxides are known to copolymerize with carbon dioxide in the presence of metal containing catalysts such as diethylzinc/additives, zinc carboxylates, and metal porphyrin complexes.

The resulting copolymers have alternating epoxide and carbon dioxide groups, and are polycarbonates. These copolymers have found uses in many areas, such as ceramic binders, evaporative pattern casting and adhesives. Epoxides shown to proceed readily in this reaction are ethylene oxide, propylene oxide, cyclohexane oxide, cis-2-butene oxide and others.

FR-A-1462491 describes the production of glycidol by the epoxidation of allyl alcohol with a hydroperoxide in an organic solvent and in the presence of a metal catalyst. The epoxidation reaction product is then distilled to recover the unreacted alcohol for recycle before extracting the residual fraction with water to recover the glycidol.

Later published US-A-3374153 refers to the process described in the aforementioned French patent publication and teaches that the separation of the reaction mixture described therein is particularly difficult because of the instability of the glycidol; i.e. the tendency of the glycidol to decompose at the temperatures required for distillation, even if the distillation is conducted at reduced pressure. The U.S. publication further teaches that while it may be possible, as an alternative, to provide pressures sufficiently low to permit obtention of low enough distillation temperatures to avoid decomposition, this is prohibitively expensive on a commercial scale. By way of improvement, the U.S. publication teaches the separation of the glycidol from the unreacted allyl alcohol by azeotropic distillation employing a hydrocarbon capable of forming a low-boiling azeotrope with glycidol. The glycidol is thereafter recovered from its azeotrope by extraction with water. The method of the U.S. patent requires that the excess hydroperoxide be destroyed by the addition of expensive trialkyl phosphites. Direct distillation of the product containing the phosphites involves certain hazards which are best avoided.

It has now been found that the addition of phosphite and the distillation step can be eliminated by direct extraction of the water-soluble glycidol with water after the epoxidation reaction, thereby providing an economical means for separating the epoxyalcohol from the catalysts and the hydroperoxide oxidate which remain in the organic phase.

According to the present invention there is provided a process for the production of a glycidol by reacting an allylic alcohol with an organic hydroperoxide in a organic solvent in the presence of a metal catalyst and wherein the recovery of the glycidol includes extracting it with water and subsequent distillation wherein the organic hydroperoxide is essentially water-insoluble, the process includes the steps of:
(a) adding water to the product mixture resulting from the reaction to form an aqueous phase and a non-aqueous phase with the glycidol concentrated in the aqueous phase;
(b) separating the aqueous phase from the non-aqueous phase;
(c) removing water from the aqueous phase; and thereafter
(d) distilling off the glycidol.

The allylic alcohols suitable for use in the epoxidation reaction may have the following general formula: 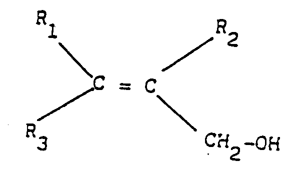
where each of R₁, R₂ and R₃, which may be the same or different, is selected from h, C₁-C₁₀ linear or branched alkyl, phenyl, and substituted phenyl. The allylic alcohol may be selected from allyl alcohol, annamyl alcohol ad methallyl alcohol.

The organic hydroperoxides useful in the invention may be aliphatic hydroperoxides wherein the aliphatic groups have from one to twenty carbon atoms. Illustrative hydroperoxides are ethylbenzene hydroperoxide, cumene hydroperoxide, tert-butylhydroperoxide, and trityl hydroperoxide. The only criteria for the hydroperoxide is that it must be essentially insoluble in water.

The metal catalysts useful in the invention include various salts of vanadium, titanium, tantalum, zirconium, hafnium, niobium ad molybdenum in conjunction with a chiral carbinol, such as diisopropyl tartrate. Particularly useful salts are the naphthenates, the isopropoxides, and other alkoxides.

The useful organic solvents, whose only limitation is the need for water-insolubility, may include hexane, toluene, tetrahydrofuran, methylene chloride, methyl acetate, and mixtures of these. The solvent also should not react with the reactants in the medium to interfere with the transfer of the oxygen atom from the oxidant to the allylic unsaturation.

The epoxidation reaction temperature may vary from - 100°C to 20°C and the times may vary from a few minutes to a few days.

The extraction of the glycidol product from the organic mixture is effected with water. Several consecutive extractions may be run to recover a maximum amount of the glycidol product.

After separating the aqueous extraction layers from the water-insoluble organic layers, the water extracts are combined and the water removed by flash distillation under suitable vacuum at about 50°C. The remaining glycidol product can then be fractionally distilled under vacuum to isolate the pure product.

The following examples are meant to further illustrate, but not to limit the invention.

### EXAMPLE I

Epoxidation of allyl alcohol using titanium isopropoxide/L(+)-diisopropyl tartrate catalyst and cumene hydroperoxide oxidate was carried out in methylene chloride in the presence of molecular sieves at -15°C for 16 hours to give 65% yield of glycidol.The molecular sieves were filtered off and 400g of this solution containing 22g of glycidol was extracted with 100g of water after stirring at 190 rpm for 5 minutes at 0°C. There was obtained 10.8g of glycidol in this first extraction. Extraction of the organic layer with an additional 100g of water yielded 5.8g more of glycidol. Glycidol recovery in two extractions was 75% of the total present in the organic mixture. The two aqueous fractions were combined and the active oxygen analyzed to be 320 ppm, indicating low cumene hydroperoxide extraction. Subsequent water removal and fractional distillation gave 99% pure glycidol. The enantiomeric excess of the (S)-glycidol made was determined to be 88% by the Mosher ester/¹³C NMR method.

### EXAMPLE II

To show that the extraction can be applied to other water-soluble epoxyalcohols, the following experiment was tried. Epoxidation of methallyl alcohol using titanium isopropoxide/L(+)-diisopropyl tartrate catalyst and cumene hydroperoxide oxidate was carried out in methylene chloride in the presence of molecular sieves at -15°C for 16 hours to give 87% yield of methyl glycidol. The molecular sieves were filtered off and 200g of this solution containing 19.1g of methyl glycidol was diluted with 204g of methylene chloride and extracted with 100g of water. There was obtained 6.2g of methyl glycidol in this first extraction. Extraction of the organic layer with an additional 100g of water yielded 4.1g more of methyl glycidol. Methyl glycidol recovery in two extractions was 54% of the total present in the organic mixture. The two aqueous fractions were combined and the active oxygen analyzed to be 247 ppm, indicating low cumene hydroperoxide extraction. Subsequent water removal and fractional distillation gave 99% pure methyl glycidol. The enantiomeric excess of the (S)-methyl glycidol made was determined to be 89% by the Mosher ester/¹³C NMR method.

## Claims

1. A process for the production of a glycidol by reacting an allylic alcohol with an organic hydroperoxide in an organic solvent in the presence of a metal catalyst and wherein the recovery of the glycidol includes extracting it with water and subsequent distillation characterised in that the organic hydroperoxide is essentially water-insoluble and the process includes the steps of:
(a) adding water to the product mixture resulting from the reaction to form an aqueous phase and a non-aqueous phase with the glycidol concentrated in the aqueous phase;
(b) separating the aqueous phase from the non-aqueous phase;
(c) removing water from the aqueous phase; and thereafter
(d) distilling off the glycidol.

2. The process of claim 1 wherein the allylic alcohol has the following general formula 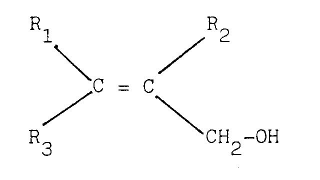 where each of R₁, R₂, and R₃, which may be the same or different is selected from H, C₁-C₁₀ linear or branched alkyl, phenyl, and substituted phenyl.

3. The process of claim 2 wherein the allylic alcohol is selected from allyl alcohol, cinnamyl alcohol, and methallyl alcohol.

4. The process of claim 1, claim 2 or claim 3 wherein the organic hydroperoxide is selected from aliphatic hydroperoxides wherein the aliphatic group has from one to 20 carbon atoms and the hydroperoxide is essentially insoluble in water.

5. The process of claim 4 wherein the hydroperoxide is selected from ethylbenzene hydroperoxide, cumene hydroperoxide, tert-butylhydroperoxide, and trityl hydroperoxide.

6. The process of any one of claims 1 to 5 wherein the metal catalyst comprises a naphthenate or alkoxide of a metal selected from the group consisting of vanadium, titanium, tantalum, zirconium, hafnium, niobium, and molybdenum used in conjunction with a chiral carbinol.

7. The process of claim 6 wherein the chiral carbinol is a dialkyl tartrate.

8. The process of any one of claims 1 to 7 wherein the organic solvent is selected from the group consisting of hexane, toluene, tetra-hydrofuran, methylene chloride, methyl acetate, and mixtures of these.

## Patentansprüche

1. Verfahren zur Herstellung von Epoxyalkoholen durch Umsetzung eines Allylalkohols mit einem organischen Hydroperoxid in einem organischen Lösungsmittel in Anwesenheit eines metallischen Katalysators, wobei die Gewinnung des Epoxyalkohols eine Extraktion mit Wasser und anschließende Destillation einschließt, dadurch gekennzeichnet, daß das organische Hydroperoxid im wesentlichen wasserunlöslich ist und man bei dem Verfahren:
(a) zu dem aus der Umsetzung erhaltenen Produktgemisch Wasser zusetzt, damit sich eine wäßrige und eine nichtwäßrige Phase bilden, wobei der Epoxyalkohol konzentriert in der wäßrigen Phase vorliegt,
(b) die wäßrige Phase von der nichtwäßrigen Phase abtrennt,
(c) das Wasser aus der wäßrigen Phase entfernt und danach
(d) den Epoxyalkohol abdestilliert.

2. Verfahren gemäß Anspruch 1, bei dem der Allylalkohol folgende allgemeine Formel aufweist 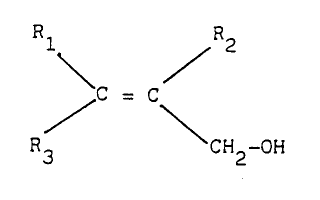 in der jeder der Reste R₁, R₂ und R₃, die gleich oder verschieden sein können, aus H, einem linearen oder verweigten C₁₋₁₀-Alkyl, Phenyl und substituiertem Phenyl ausgewählt ist.

3. Verfahren gemäß Anspruch 2, bei dem der Allylalkohol aus Allylalkohol, Cinnamylalkohol und Methallylalkohol ausgewählt ist.

4. Verfahren gemäß Anspruch 1, 2 oder 3, bei dem das organische Hydroperoxid aus aliphatischen Hydroperoxiden ausgewählt ist, deren aliphatischer Rest 1 bis 20 Kohlenstoffatome aufweist, wobei das Hydroperoxid im wesentlichen wasserunlöslich ist.

5. Verfahren gemäß Anspruch 4, bei dem das Hydroperoxid aus Ethylbenzolhydroperoxid, Cumolhydroperoxid, t-Butylhydroperoxid und Tritylhydroperoxid (Triphenylhydroperoxid) ausgewählt ist.

6. Verfahren gemäß einem der Ansprüche 1 bis 5, bei dem der metallische Katalysator ein Naphthenat oder Alkoxid eines Metalls enthält, das aus der aus Vanadium, Titan, Tantal, Zirconium, Hafnium, Niob und Molybdän bestehenden Gruppe ausgewählt ist und in Verbindung mit einem chiralen Carbinol eingesetzt wird.

7. Verfahren gemäß Anspruch 6, bei dem das chirale Carbinol ein Dialkyltartrat darstellt.

8. Verfahren gemäßeinem der Ansprüche 1 - 7, bei dem das organische Lösungsmittel aus der aus Hexan, Toluol, Tetrahydrofuran, Methylenchlorid, Methylacetat und Gemischen derselben bestehenden Gruppe ausgewählt ist.

## Revendications

1. Procédé pour la production d'un glycidol par réaction d'un alcool allylique avec un hydroperoxyde organique dans un solvant organique en présence d'un catalyseur métal, et dans lequel la récupération du glycidol comprend l'extraction de ce dernier par l'eau, suivie d'une distillation caractérisée en ce que l'hydroperoxyde organique est essetiellement insoluble dans l'eau, procédé qui comprend les étapes:
(a) d'addition d'eau au mélange contenant le produit obtenu à partir de la réaction, pour former une phase aqueuse, et une phase non aqueuse, le glycidol étant concentré dans la phase aqueuse;
(b) de séparation de la phase aqueuse et de la phase non aqueuse;
(c) d'elimination de l'eau de la phase aqueuse; et ensuite,
(d) de séparation du glycidol par distillation.

2. Procédé selon la revendication 1, dans lequel l'alcool allylique a la formule générale suivante : 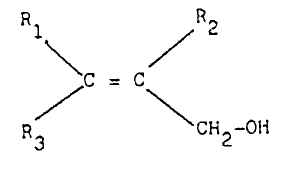 dans laquelle chacun des groupes R₁, R₂, et R₃ qui peuvent être identiques ou différents, est choisi parmi l'atome d'hydrogène, les groupes alkyle C₁-C₁₀, linéaires ou ramifiés, phényle, et phényle substitué.

3. Procédé selon la revendication 2, dans lequel l'alcool allylique est choisi parmi l'alcool allylique l'alcool cinnamique, ou l'alcool méthallylique.

4. Procédé selon la revendication 1, la revendication 2, ou la revendication 3, dans lequel l'hydroperoxyde organique est choisi parmi des hydroperoxydes aliphatiques dans lesquels le groupe aliphatique comporte 1 à 20 atomes de carbone, et l'hydroperoxyde est essentiellement insoluble dans l'eau.

5. Procédé selon la revendication 4, dans lequel l'hydroperoxyde est choisi parmi l'hydroperoxyde d'éthylbezène, l'hydroperoxyde de cumène, l'hydroperoxyde de ter-butyle, et l'hydroperoxyde de trityle.

6. Procédé selon l'une quelconque des revendications 1 à 5, dans lequel le catalyseur métal comprend un naphténate ou un alcoxyde d'un métal choisi au sein du groupe comprenant le vanadium, le titane, le zirconium, le hafnium, le niobium, et le molybdène utilisé en conjonction avec un carbinol chiral.

7. Procédé selon la revendication 6, dans lequel le carbinol chiral est un tartrate de dialkyle.

8. Procédé selon l'une quelconque des revendications 1 à 7, dans lequel le solvant organique est choisi au sein du groupe comprenant l'hexane, le toluène, le tétrahydrofurane, le dichlorométhane, l'acétate de méthyle, et les mélanges de ceux-ci.
